# EUROPÄISCHE PATENTANMELDUNG

(11) **EP 2 165 670 A1**
(43) Veröffentlichungstag der Anmeldung: **24.03.2010**
(21) Anmeldenummer: 08164700.0
(22) Anmeldetag: 19.09.2008
(51) Int. Cl.: A61B 19/00, H01L 31/048

(54) **Medizinisches Instrument mit solarzellengestützter Energieversorgung**

(71) Anmelder: BrainLAB AG, 85622 Feldkirchen (DE)
(72) Erfinder: Urban, Alexander, 85570 Markt Schwaben (DE); Hager, Stefan, 80687 München (DE)
(74) Vertreter: Schwabe - Sandmair - Marx

(57) **Zusammenfassung**

Die Erfindung betrifft ein medizintechnisches Instrument (1) mit zumindest einem Energieverbraucher (2) und einer Energieversorgungseinrichtung (3), wobei die Energieversorgungseinrichtung (3) zumindest ein Wandlerelement (4) umfasst, das Strahlungsleistung in elektrische Leistung wandelt.

## Beschreibung

Die Erfindung betrifft ein medizintechnisches Instrument mit solarzellengestützter Energieversorgung. Sie betrifft insbesondere medizintechnische Instrumente, die einen Energieverbraucher aufweisen, wobei die Energieversorgung des Energieverbrauchers durch Vorsehen eines Wandlerelements sichergestellt wird, das Strahlungsleistung in elektrische Leistung wandelt.

In der computergestützten Chirurgie werden dem Behandelnden zusehends öfter operationsunterstützende Vorrichtungen zur Verfügung gestellt, beispielsweise Navigations- bzw. Trackingsysteme im Operationssaal. Mit Hilfe solcher Systeme können etwa Instrumente oder Behandlungseinrichtungen im Positionsverhältnis zum Patienten angezeigt werden, um so dem Behandelnden eine visuelle Unterstützung zu geben.

Je nach Aufgabe der unterschiedlichen medizintechnischen Instrumente ist es oftmals notwendig, einzelne oder alle medizintechnischen Instrumente mit einer eigenen Energieversorgung auszustatten. Dies ist beispielsweise dann der Fall, wenn die Instrumente eigene Anzeigen haben, welche den Behandelnden auf bestimmte Zustände hinweisen, das Instrument aktive Sensoren umfasst, oder auch dann, wenn das Instrument aktive Marker zur Verfolgung und Erkennung des Instruments durch ein medizintechnisches Navigation- bzw. Trackingsystem umfasst.

Im Stand der Technik wurde dieses Problem bisher so gelöst, dass das Instrument über Energieversorgungskabel oder Drähte mit einer externen Energieversorgungseinrichtung verbunden wurde. Ein Nachteil dieser Lösung besteht sicherlich darin, dass der Behandelnde durch solche Drähte oder Kabel bei seiner Tätigkeit gestört, wenn nicht sogar behindert wird.

Um eine solche Behinderung zu vermeiden, werden medizintechnische Instrumente oftmals mit einer eigenen Energieversorgung in Form von Batterien oder Akkumulatoren ausgestattet. In diesem Falle gestaltet sich die Sterilisation solcher Instrumente ziemlich schwierig, da die verwendeten Batterien oder Akkumulatoren den hohen Temperaturen bei einer Autoklaven-Sterilisation nicht standhalten. So müssen diese vor der Sterilisation aus dem Instrument ausgebaut werden, was zusätzlichen Aufwand bedeutet. Da für unterschiedliche Instrumente oftmals unterschiedliche Typen von Batterien bzw. Akkumulatoren verwendet werden, besteht hierbei wiederum die Gefahr, dass einzelne Batterien bzw. Akkumulatoren vertauscht werden und in die falschen Instrumente eingesetzt werden, so dass Fehlfunktionen oder sogar Beschädigungen einzelner Instrumente auftreten können.

Ein weiterer Nachteil von batterie- bzw. akkumulatorenbetriebenen Instrumenten besteht in deren hohem Gewicht, was die Handhabbarkeit negativ beeinträchtigt und letztendlich die Akzeptanz solcher Instrumente schmälert.

Es ist die Aufgabe der vorliegenden Erfindung, ein medizintechnisches Instrument mit einem Energieverbraucher bereitzustellen, welches auf einfache und unkomplizierte Weise sterilisiert werden kann und trotzdem eine gute Handhabbarkeit aufweist.

Diese Aufgabe wird durch ein medizintechnisches Instrument gemäß dem Anspruch 1 gelöst. Die Unteransprüche definieren dabei bevorzugte Ausführungsformen der vorliegenden Erfindung.

Das erfindungsgemäße medizintechnische Instrument weist zumindest einen Energieverbraucher und eine Energieversorgungseinrichtung auf, welche den Energieverbraucher mit elektrischer Leistung versorgt. Dabei umfasst die Energieversorgungseinrichtung zumindest ein Wandlerelement, das Strahlungsleistung in elektrische Leistung umwandelt, die der Energieverbraucher benötigt.

In einer bevorzugten Ausführungsform ist das Wandlerelement eine Solarzelle, die natürliches oder künstliches Licht, insbesondere direkt in elektrische Leistung wandelt, obwohl grundsätzlich die Wandlung jeglicher elektromagnetischer Strahlungsleistung denkbar wäre. Da bei medizinischen Operationen am Operationsort stets für eine sehr gute Beleuchtung gesorgt werden muss, steht für die Solarzelle oder Solarzellen des Instruments immer genügend Strahlungsleistung bereit. Gegenüber herkömmlichen batteriebetriebenen Instrumenten ist an dieser Stelle ein weiterer Vorteil der vorliegenden Erfindung zu nennen: Sobald Licht auf die Solarzellen des Instruments fällt, also nachdem die Operationssaalbeleuchtung eingeschaltet wurde und die Instrumente aus ihrer Verpackung genommen wurden, stehen sie sofort zur Verfügung und müssen nicht wie herkömmliche batteriebetriebene Instrumente in langen Aufladezyklen geladen werden. Auch sind Solarzellen resistent gegen die bei einer Autoklaven-Sterilisation auftretenden Temperaturen und können daher dabei grundsätzlich am oder im Instrument verbleiben.

Um dem Energieverbraucher des Instruments stets nur die benötigte elektrische Leistung bereitzustellen, kann die Energieversorgungseinrichtung zusätzlich eine Energiespeisung umfassen, die elektrische Leistung vom Wandlerelement empfängt und elektrische Leistung in definierter Weise an den Energieverbraucher abgibt. So kann der Energieverbraucher bei sehr hoher Strahlungsleistung immer mit der von ihm benötigten elektrischen Leistung versorgt werden, ohne dass eine Zerstörung des Energieverbrauchers durch zu hohe elektrische Leistung befürchtet werden muss.

Weiterhin ist es denkbar, dass die Energiespeisung elektrische Energie speichern bzw. zwischenspeichern kann, um beispielsweise in Zeiten sehr hoher Strahlungsleistung die überschüssige gewandelte elektrische Energie zwischenzuspeichern um diese dann zu Zeitpunkten an den Energieverbraucher abzugeben, wenn dieser mehr elektrische Leistung benötigt, als momentan vom Wandlerelement abgegeben werden kann.

Als Energieverbraucher sind insbesondere Navigations- bzw. Kommunikationseinrichtungen (wie etwa Bluetooth, WiFi, LED) für ein Navigations- bzw. Trackingsystem, aktive Marker oder aktive Sensoren denkbar, jedoch auch Anzeigen, die den Behandelnden mit Information versorgen, wie etwa LED- oder Flüssigkristallanzeigen. Es ist also möglich, dass ein einziges Instrument mehrere Energieverbraucher umfassen kann, die alle von der Energieversorgungseinrichtung elektrische Leistung beziehen. Auch ist eine Anzeige denkbar, welche den Behandelnden über den momentanen Energiestatus des Instruments informiert, wie etwa den Ladezustand des Energiespeichers bzw. Kondensators, den momentanen Leistungsverbrauch oder die momentan vom Wandlerelement abgegebene elektrische Leistung.

In einer weiteren bevorzugten Ausführungsform ist das Wandlerelement im Bereich des medizinischen Instruments oder an diesem angeordnet, so dass die zumindest auf einen Teil der Oberfläche des Instruments auftreffende Strahlungsleistung in elektrische Leistung gewandelt wird. Insbesondere kann das Wandlerelement auf der Oberfläche des Instruments angeordnet sein. Vorteilhafterweise ist das Wandlerelement direkt am Griffstück bzw. an der Griffstückoberfläche angeordnet.

Jedoch ist es auch denkbar, dass das Wandlerelement in den Instrumentenkörper eingelassen ist, also gegenüber der Instrumentenoberfläche zurückversetzt ist. In diesem Falle kann das Wandlerelement besonders gut durch eine Abdeckung von schädlichen äußeren Einflüssen geschützt werden, während die Oberfläche der Abdeckung plan mit der Instrumentenoberfläche abschließt. Bei Operationen ist es insbesondere von Vorteil, wenn die Abdeckung zusätzlich schmutzabweisende Eigenschaften aufweist, so dass immer für eine maximale Abgabe elektrischer Leistung gesorgt ist. Dazu kann die Abdeckung eine Nanobeschichtung aufweisen, die durch den sogenannten Lotusblüteneffekt Schmutz und Flüssigkeiten sofort von der Abdeckungsoberfläche abgleiten oder zumindest diese leichter entfernen lässt.

Um die Instrumentenlebensdauer unabhängig von der Lebensdauer des Wandlerelements bzw. der Energieversorgungseinrichtung zu machen, kann das Wandlerelement und insbesondere die gesamte Energieversorgungseinrichtung vom Instrument abnehmbar ausgestaltet sein, so dass im Falle einer Beschädigung einzelner Bauelemente diese leicht ersetzt werden können, ohne dass das gesamte Instrument entsorgt werden muss.

Für den Fall, dass das medizintechnische Instrument Marker für ein medizintechnisches Navigation- bzw. Trackingsystem aufweist, ist es von Vorteil, wenn das Wandlerelement im Wesentlichen an der Instrumentenseite angeordnet ist, an der auch die Marker angeordnet sind. Da zumindest optische Marker eine Sichtlinie zu den Sensoren des Navigations- bzw. Trackingsystems haben müssen und deshalb auch dafür gesorgt wird, dass diese Sichtlinie so wenig wie möglich unterbrochen wird, bietet es sich geradezu an, sich dieses Bemühen auch bei der Bestrahlung des Wandlerelements zu Nutze zu machen. Die Sensoren des Navigations- bzw. Trackingsystems sind nämlich meistens im Wesentlichen oberhalb des Operationsgeschehens angeordnet, wie ebenfalls auch die Operationslampe. So kann dann davon ausgegangen werden, dass bei einer freien Sichtlinie zwischen Marker und Navigation- bzw. Trackingsystem auch für eine einwandfreie Bestrahlung des Wandlerelements gesorgt ist.

In Verbindung mit dem erfindungsgemäßen medizintechnischen Instrument besteht die Möglichkeit, das gesamte Instrument zumindest zu einem Großteil aus einem nichtmagnetischen Material zu fertigen. So weist ein Kondensator grundsätzlich weniger metallhaltige Bauteile auf als Batterien bzw. Akkumulatoren. Ferner können die Kondensatorplatten eines gegebenenfalls verwendeten Kondensators lediglich aus einem mit Metall bedampften Kunststoff bestehen. Letztendlich kann das erfindungsgemäße Instrument durch Reduktion magnetischer Bauteile so ausgestaltet werden, dass es auf dem Gebiet der MR-Technologie eingesetzt werden kann, während herkömmliche Instrumente durch ihre metallhaltigen Batterien bzw. Akkumulatoren dafür nicht geeignet sind.

Bisher wurde der Begriff medizintechnisches Instrument pauschal für alle Instrumente und Geräte verwendet, die einen Energieverbraucher mit einem Leistungsbedarf aufweisen, der durch in ausreichender Zahl vorhandener Batterien, Akkumulatoren oder Strahlungsenergiewandler wie Solarzellen gedeckt werden kann. Solche Instrumente oder Geräte sind z.B. jegliche in der Hand gehaltene und geführte Instrumente wie medizintechnische Pointer, Softtouch-Pointer oder Z-touch, jedoch auch statische oder bewegliche Geräte, die während eines längeren Zeitraums an Ort und Stelle verbleiben oder sich bewegen, wie beispielsweise Knochenschneidblöcke, Referenzeinrichtungen oder aktive Referenzmarker und jegliche Art von Anzeigen, wie etwa LCD-Bildschirme.

Da in der Hand gehaltene Instrumente meistens in einer bevorzugten Position und Ausrichtung in der Hand gehalten werden, ist es von Vorteil, wenn das Wandlerelement von der an der Hand anliegenden Instrumentenseite weg weist und somit zu wandelnde Strahlungsleistung empfangen kann, ohne von der Hand verdeckt zu werden.

Um sicherzustellen, dass sich unabhängig von der Lage und Position des Instruments in der Hand das eine bzw. eines von mehreren Wandlerelementen in direkter Sichtlinie zu einer Strahlungsquelle bzw. Beleuchtungseinrichtung wie einer Operationslampe befindet, ist es denkbar, dass im Wesentlichen der gesamte Umfang des Instruments mit einem oder mehreren Wandlerelementen ausgestattet ist. So ist im Speziellen ein ringförmiger Bereich in Umfangsrichtung des Instruments denkbar, in dem Wandlerelemente angeordnet sind und unabhängig von der Lage und Ausrichtung des Instruments stets elektrische Leistung an den Energieverbraucher abgeben können.

Die Erfindung wird im Weiteren anhand einer Ausführungsform näher erläutert. Sie kann alle hierin beschriebenen Merkmale einzeln oder in jedweder sinnvollen Kombination umfassen.

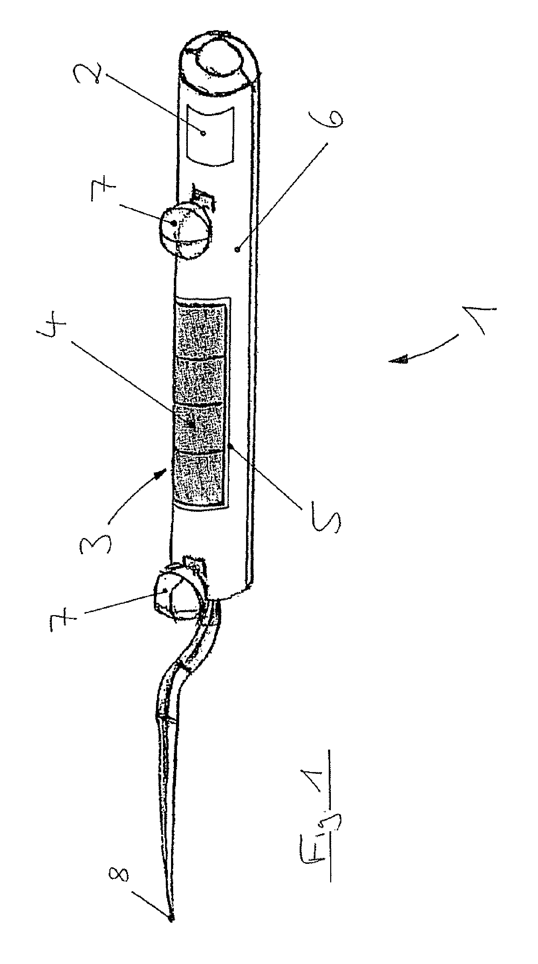

In der einzigen beiliegenden Figur 1 ist ein erfindungsgemäßes medizintechnisches Instrument mit einer Energieversorgungseinrichtung gezeigt, die wiederum ein Wandlerelement umfasst.

Grundsätzlich umfasst das erfindungsgemäße medizintechnische Instrument 1 einen Energieverbraucher 2 und eine Energieversorgungseinrichtung 3. In der gezeigten Ausführungsform ist der Energieverbraucher 2 eine Anzeige, welche den Behandelnden darüber informiert, ob das Instrument 1, hier ein medizintechnischer Softtouch-Pointer mit seiner in der Figur 1 nach links gerichteten aktiven Sensorspitze 8 die Oberfläche des Patienten berührt. Zur Energieversorgung der Anzeige 2 und des Sensors 8 sind am Griffstück 6 vier Wandlerelemente 4 in Form von Solarzellen angeordnet. Der Sensor 8 ist bei der gezeigten Ausführungsform ein kapazitives Element, welche auf die Berührung der Sensorspitze mit dem Patienten reagiert.

Diese vier Solarzellen 4 bilden zusammen mit einem im Inneren des Griffstücks 6 und nicht gezeigten Kondensator die Energieversorgungseinrichtung 3. Der Kondensator stellt als eine Art "Energiepuffer" die benötigte Energieversorgung der Anzeige 2 sicher, selbst wenn die Solarzellen 4 verdeckt werden sollten.

Weiterhin sind die Solarzellen 4 von einer nanobeschichteten Abdeckung 5 geschützt, die sich über die gesamte Ausdehnung der Solarzellen 4 erstreckt und mit ihrer Oberfläche plan mit der Oberfläche des Griffstücks 6 abschließt. Durch den planen Abschluss wird die Handhabbarkeit des Pointers 1 erleichtert. Ferner wird durch die Nanobeschichtung der Abdeckung 5 ein Lotusblüteneffekt erzielt, so dass sich Schmutz oder Flüssigkeiten auf der Abdeckung 5 leicht durch Abwischen entfernen lassen, sofern diese nicht ohnehin sofort von der Oberfläche der Abdeckung 5 abgleiten. So wird stets der maximal mögliche Eintrag von Strahlungsleistung durch die Abdeckung 5 auf die Solarzellen 4 sichergestellt.

Ferner sind die Solarzellen 4 zwischen zwei Markern 7 angeordnet. Da der Behandelnde den Pointer 1 ohnehin immer so halten würde, dass die Marker 7 nicht durch seine Hand oder seinen Körper relativ zu den Sensoren des Navigations- bzw. Trackingsystems verdeckt werden würden, kann bei dieser Anordnung der Solarzellen 4 zwischen den Markern 7 vorteilhafterweise immer davon ausgegangen werden, dass diese ebenso frei von jeder Abdeckung durch die Hand oder Finger des Behandelnden sind.

In Verbindung mit der vorliegenden Erfindung werden keine Ladestationen benötigt und es besteht nie die Gefahr, dass wie bei batteriegetriebenen Instrumenten diese aufgrund eines niedrigen Ladezustands nicht einsatzfähig sind. Ferner kann auch auf gefährliche Chemikalien oder Stoffe im Operationsraum verzichtet werden, die in Batterien oder Akkumulatoren vorhanden sind

## Patentansprüche

1. Medizintechnisches Instrument (1) mit zumindest einem Energieverbraucher (2) und einer Energieversorgungseinrichtung (3), **dadurch gekennzeichnet, dass** die Energieversorgungseinrichtung (3) zumindest ein Wandlerelement (4) umfasst, das Strahlungsleistung in elektrische Leistung wandelt.

2. Medizintechnisches Instrument nach Anspruch 1, **dadurch gekennzeichnet, dass** das Wandlerelement (4) eine Solarzelle ist, die natürliches oder künstliches Licht insbesondere direkt in elektrische Leistung wandelt.

3. Medizintechnisches Instrument nach Anspruch 1 oder 2, **dadurch gekennzeichnet, dass** die Energieversorgungseinrichtung (3) eine Energiespeisung umfasst, die elektrische Leistung vom Wandlerelement (4) empfängt und elektrische Leistung in definierter Weise an den zumindest einen Energieverbraucher (2) abgibt.

4. Medizintechnisches Instrument nach Anspruch 3, **dadurch gekennzeichnet, dass** die Energiespeisung elektrische Leistung speichert, insbesondere in einem Kondensator speichert und bei Bedarf an den zumindest einen Energieverbraucher (2) abgibt.

5. Medizintechnisches Instrument nach einem der Ansprüche 1 bis 4, **dadurch gekennzeichnet, dass** das Wandlerelement (4) die auf zumindest einen Teil der Oberfläche des medizintechnischen Instruments (1) auftreffende Strahlungsleistung wandelt.

6. Medizintechnisches Instrument nach einem der Ansprüche 1 bis 5, **dadurch gekennzeichnet, dass** das Wandlerelement (4) auf oder zumindest im Bereich der Instrumentenoberfläche, insbesondere am Griffstück (6) des Instruments (1) angeordnet ist.

7. Medizintechnisches Instrument nach einem der Ansprüche 1 bis 6, **dadurch gekennzeichnet, dass** das Wandlerelement (4) unter einer strahlungsdurchlässigen und insbesondere schmutzabweisenden, im Speziellen nanobeschichteten Abdeckung (5) angeordnet ist.

8. Medizintechnisches Instrument nach einem der Ansprüche 1 bis 7, **dadurch gekennzeichnet, dass** das Wandlerelement (4) und insbesondere die Energieversorgungseinrichtung (3) vom Instrument (1) abnehmbar, insbesondere auswechselbar ausgestaltet ist.

9. Medizintechnisches Instrument nach einem der Ansprüche 1 bis 8 mit Markern (7) für ein medizintechnisches Navigations- bzw. Trackingsystem, **dadurch gekennzeichnet, dass** das Wandlerelement (4) im Wesentlichen an der Instrumentenseite angeordnet ist, an der auch die Marker (7) angeordnet sind.

10. Medizintechnisches Instrument nach einem der Ansprüche 1 bis 9, **dadurch gekennzeichnet, dass** das Instrument (1), insbesondere das Instrumentengehäuse im Wesentlichen aus einem nichtmagnetischen Material, insbesondere einem nichtmagnetischen Kunststoff gefertigt ist, so dass das Instrument (1) auf dem Gebiet der MR-Technologie eingesetzt werden kann.

11. Medizintechnisches Instrument nach einem der Ansprüche 1 bis 10, **dadurch gekennzeichnet, dass** das Instrument (1) ein in der Hand gehaltenes Instrument ist, insbesondere ein medizintechnischer Pointer ist.

12. Medizintechnisches Instrument nach Anspruch 11, **dadurch gekennzeichnet, dass** das Wandlerelement (4) beim in der Hand gehaltenen Instrument (1) von der an der Hand anliegenden Instrumentenseite weg weist oder sich im Wesentlichen über den gesamten radialen Umfang des Instruments (1) erstreckt, insbesondere ringförmig erstreckt.

13. Medizintechnisches Instrument nach einem der Ansprüche 11 oder 12, **dadurch gekennzeichnet, dass** das Instrument (1) eine berührungsempfindliche Spitze (8) und/oder einen Sender für die Übertragung eines Berührungssignals umfasst.

14. Medizintechnisches Instrument nach einem der Ansprüche 1 bis 13, **dadurch gekennzeichnet, dass** das Instrument (1) Lichtabstrahler, insbesondere aktive Marker für ein optisches Tracking- bzw. Navigationssystem und/oder eine Lichtprojektionsvorrichtung umfasst.

15. Medizintechnisches Instrument nach einem der Ansprüche 1 bis 14, **dadurch gekennzeichnet, dass** das Instrument (1) eine optische und/oder akustische Anzeigevorrichtung, insbesondere Anzeigeflächen und/oder Lautsprecher umfasst, um dem Behandelnden Informationen bereitzustellen.
